# EUROPEAN PATENT SPECIFICATION

(11) **EP 2 772 278 B1**
(45) Date of publication and mention of the grant of the patent: **02.08.2017**
(21) Application number: 12844127.6
(22) Date of filing: 25.10.2012
(51) Int. Cl.: A61M 21/02, H05B 37/02

(54) **LIGHT EXPOSURE DEVICE FOR IMPROVING COGNITIVE SYMPTOMS AND DEPRESSION SYMPTOMS**
LICHTBESTRAHLUNGSVORRICHTUNG ZUR VERBESSERUNG VON KOGNITIVEN SYMPTONEN UND DEPRESSIONSSYMPTOMEN
DISPOSITIF D'EXPOSITION À LA LUMIÈRE POUR AMÉLIORER LES SYMPTÔMES COGNITIFS ET LES SYMPTÔMES DE DÉPRESSION

(30) Priority: 25.10.2011 JP 2011234026
(43) Date of publication of application: 03.09.2014
(73) Proprietor: Kanazawa Medical University, Uchinada-machi Kahoku-gun Ishikawa 920-0293 (JP)
(72) Inventor: KATO, Nobuo, Kahoku-gun Ishikawa 920-0293 (JP)
(74) Representative: Körfer, Thomas
(86) International application number: PCT/JP2012/006857
(87) International publication number: WO 2013/061597

(56) References cited:
- WO-A1-91/06078
- WO-A1-2008/147958
- WO-A1-2009/023968
- WO-A1-2010/122446
- WO-A1-2010/139480
- JP-A- H0 515 596
- JP-A- 2001 149 478
- US-A- 4 892 106
- US-A- 5 092 669
- US-A1- 2006 106 276
- HECHT S ET AL: "INTERMITTENT STIMULATION BY LIGHT : VI. AREA AND THE RELATION BETWEEN CRITICAL FREQUENCY AND INTENSITY.", THE JOURNAL OF GENERAL PHYSIOLOGY 20 JUL 1936, vol. 19, no. 6, 20 July 1936 (1936-07-20), pages 979-989, XP002738504, ISSN: 0022-1295

## Description

### [TECHNICAL FIELD]

The present invention relates to a light irradiation device capable of improving cognitive symptom and depression symptom, a room having this light irradiation device, and a lighting device capable of improving cognitive symptom and depression symptom.

### [BACKGROUND TECHNIQUE]

As an initial treatment method of depression, there is cognitive behavior therapy (improvement of thought pattern) which does not use medicines or a treatment instrument, but it is not always true that an effect is always exerted. For reliable treatment, medicine therapy is used. However, a success rate of treatment using only one medical agent is less than 50%. More than 50% of successful persons use many kinds of medical agents or are resistant to medical agents (non-patent document 1).

In the case of severe depression, e.g., drug-resistant depression or depression having strong suicidal thinking, electroconvulsive therapy (ECT) is used. An effect of this treatment is widely recognized between psychiatrists. In recent years, repetitive transcranial magnetic stimulation (rTMS) is growing. The repetitive transcranial magnetic stimulation is similar to electroconvulsive therapy, but the repetitive transcranial magnetic stimulation is minimally-invasive and does not cause convulsion (non-patent document 2).

The electroconvulsive therapy has merits that a treatment effect is excellent, and this is effective for drug-resistant depression. However, the electroconvulsive therapy requires not only a psychiatrist but also an anesthesiologist. Patients desire the electroconvulsive therapy, but this therapy historically has negative image. Further, the electroconvulsive therapy brings about side effects such as amnesia and cardiac damage. Concerning mechanism of the electroconvulsive therapy, there are molecular biological research (non-patent document 3) and electrophysiological research (non-patent document 4), but the mechanism is not well understood.

The repetitive transcranial magnetic stimulation does not require an anesthesiologist. The repetitive transcranial magnetic stimulation has merits that it is more minimally-invasive than the electroconvulsive therapy, and it is easier to control side effects than the electroconvulsive therapy. However, its device is expensive and although it is minimally-invasive, it is necessary to treat in clinical institutions. Although side effects are low, there still remains a possibility of induction of epilepsia, and effect of the repetitive transcranial magnetic stimulation is equal to or lower than that of the electroconvulsive therapy (non-patent document 5).

The present inventors proceeded with function analysis concerning intracellular protein Homer 1a molecules in which expression of group of molecules reaches six times by electroconvulsive stimulation used for the depression treatment. The inventors found that intracellular protein Homer 1a molecules promoted activity of BK-type potassium channel (BK channel) in pyramidal cell of cerebral cortex. The BK channel takes a role in adjusting entire activity of neuron by controlling duration of action potential, and the BK channel is important channel molecule (Sakagami et al. , Eur J Neurosci, 2005; Kato, Neurosci Res, 2009).

On the other hand, the inventors found that recently spotlighted intracellular amyloid in association with initial cognitive impairment of Alzheimer disease suppressed the same BK channel. That is, the BK channel receives adjustment in an antagonistic manner that the BK channel is activated by intracellular protein Homer 1a and is suppressed by amyloid (Yamamoto et al., J Neurosci, 2011).

This antagonistic adjustment exists. This means there is a possibility that if the intracellular protein Homer 1a is expressed, it is possible to suppress at least a portion of action of amyloid, and improvement of cognitive function can be expected. To prove this possibility, cognitive improvement achieved by expression of intracellular protein Homer 1a was investigated using an Alzheimer disease model mouse. Using Morris water maze method which is an established law for learning cognitive behavior, a defect of cognitive learning was made clear in an Alzheimer disease model mouse of three months old (announced orally at U.S. Society for Neuroscience in Neuroscience 2010).

It is clinically pointed out that it is difficult to distinguish between depression symptom and cognitive symptom. In recent years, it was epidemiologically demonstrated by a research group of Kanazawa Medical University that advancement of "disordered mood spectrum" and "cognitive impairment spectrum (order from forgetfulness to dementia through mild cognitive impairment) " of aged person in underpopulated areas is mutual serious risk factors (Private University Strategic Research Project "Research on stress coping in aging underpopulated areas" in 2008 to 2010).

Individual treatment has been applied to spiral mutual exacerbation of "disordered mood spectrum" and "dementia spectrum". When it is difficult to distinguish them, it is desirable to use multiple treatment medicine or treatment device, but such means does not exist.

Patent document 1 includes a light source for supplying stimulating pulse. There is proposed a method for treating Parkinson disease, Alzheimer disease and depression by giving stimulation to a predetermined portion using this light source.

There is a high lighting intensity light therapy used for seasonal depression. Because depression becomes worse when day time is reduced, this therapy is used for compensating the reduced time by continuously irradiating a patient with high lighting intensity light (5,000 to 10,000 lux) for one to two hours every day. In DSM-IV classification, there is no disease classification called "seasonal depression", but this means seasonal depression which resolves in summer. In this case, the patient has characteristics that daily variation pattern of blood concentration of melatonin is changed in winter, and excretion time prolongs. It is said that it is a biological objective of light irradiation to stop the prolongation of excretion (non-patent document 6).

In the case of a night time workers, it is considered that stress weakness is increased by abnormality of hypothalamic-pituitary hormonal secretion caused by disorder in daily rhythm. Therefore, it is said that it is action mechanism to act on daily disorder from facets of living by irradiating a patient with high brightness light.

It is reported that high lighting intensity light therapy is effective also for nonseasonal depression. However, it is considered that most of them are not sufficiently planned and calculated research reports (non-patent document 7) or they have slight improvement effects when it is additionally used to anti-depression medicine (non-patent document 8).

### [PRIOR ART DOCUMENT]

### [PATENT DOCUMENT]

[Patent Document 1] Japanese Translation of PCT International Application, Publication No.2008-520280

### [NON-PATENT DOCUMENTS]

[Non-Patent Document 1] Rush AJ, Fava M, Wisniewski SR et al. (2004) Sequenced treatment alternatives to relieve depression (STAR*D): rationale and design. Controlled Clinical Trials 25:119-142.
[Non-Patent Document 2] O'Reardon JP, Solvason HB, Janicak PG, Sampson S, Isenberg KE, Nahas Z, McDonald WM, Avery D, Fitzgerald PB, Loo C, Demitrack MA, George MS, Sackeim HA (2007) Efficacy and safety of transcranial magnetic stimulation in the acute treatment of major depression: a multisite randomized controlled trial. Biol Psychiatry 62:1208-1216.
[Non-Patent Document 3] Altar CA, Laeng P, Jurata LW, Brockman JA, Lemire A, Bullard J, Bukhman YV, Young TA, Charles V, Palfreyman MG (2004) Electroconvulsive seizures regulate gene expression of distinct neurotrophic signaling pathways. J Neurosci 24:2667-2677.
[Non-Patent Document 4] Kato N (2009) Neurophysiological mechanisms of electroconvulsive therapy for depression. Neurosci Res 64:3-11.
[Non-Patent Document 5] Eranti S, Mogg A, Pluck G, et al. (2007). "A randomized, controlled trial with 6-month follow-up of repetitive transcranial magnetic stimulation and electroconvulsive therapy for severe depression". Am J Psychiatry 164: 73-81.
[Non-Patent Document 6] Provencio I (2005) Chronobiology. In Kaplan & Sadock's comprehensive textbook of psychiatry 8th Ed. Sadock, BJ and Sadock, VZ, Eds. pp169-171. Lippincott Williams & Wilkins. Philadelphia, USA.
[Non-Patent Document 7] Provencio I (2005) Chronobiology. In Kaplan & Sadock's comprehensive textbook of psychiatry 8th Ed. Sadock, BJ and Sadock, VZ, Eds. pp169-171. Lippincott Williams & Wilkins. Philadelphia, USA.
[Non-Patent Document 8] Kripke DF (1998) Light treatment for nonseasonal depression: speed, efficacy, and combined treatment. J Affective Disorders 49: 109-117.

U.S.patent application US2006/0106276 discloses a device for inducing a relaxation state in a user, the device comprising a first light source, a second light source and a flash frequency control allowing the light sources to flash at a low frequency between 1 and 7.5 Hz or high frequency of over 100Hz.

International patent application WO2009/023968 discloses a device for influencing a condition in a subject, the device comprising a first light source, a second light source and flash frequency controls allowing to independently adjust the first and second light sources to flash at a frequency between 10Hz and 10kHz.

### [SUMMARY OF THE INVENTION]

### [PROBLEM TO BE SOLVED BY THE INVENTION]

From a result of research of the present inventors, if two devices, i.e., an electroconvusion stimulation device and a transcranial magnetic stimulation device are used, a possibility that a composite treatment of Alzheimer disease and depression can be provided is high.

However, the electroconvusion stimulation device and the transcranial magnetic stimulation device can be used only in clinical institutions. A convenience degree is low, and burden of expense for beneficiary and society is large.

If photostimulation is given, expression of acute and evanescent intracellular protein Homer 1a is increased for five to six hours. However, only by the photostimulation that does not accompany Homer 1a increase, improvement of cognitive learning can not be found at all.

Patent document 1 utilizes stimulating pulse by a light source but does not express intracellular protein Homer 1a. Patent document 1 implants a light-emitting device in a patient body using laser as the light source.

Hence, it is an object of the present invention to exert an effect equivalent to a depression symptom improving effect obtained by an electroconvusion stimulation device, an effect equivalent to a depression symptom improving effect and an effect equivalent to a cognitive symptom improving effect obtained by a transcranial magnetic stimulation device. It is also an object of the invention to utilize these devices also in home environment as compared with an electroconvusion stimulation device and a transcranial magnetic stimulation device which are limited to treatment in clinical institutions as opposed to home environment.

### [MEANS FOR SOLVING THE PROBLEM]

The invention is defined in the appended claim 1. In a light irradiation device for improving cognitive symptom and depression symptom according to a first aspect, photostimulation is given to a retina by continuous flashing of a light source of frequency of 1 to 10 Hz.

According to a second aspect, in the light irradiation device for improving cognitive symptom and depression symptom of the first aspect, Homer 1a is made to continuously express by the photostimulation, and BK-type potassium channel in pyramidal cell of cerebral cortex is activated.

A room of a third aspect includes the light irradiation device for improving cognitive symptom and depression symptom according to the first or second aspect.

A fourth aspect provides a lighting device for improving cognitive symptom and depression symptom including a light source, frequency changing means for changing frequency of the light source, an irradiation switch for changing the frequency to high frequency of 20 to 50 kHz by the frequency changing means to make the light source illuminate, and a light-irradiation switch for changing the frequency to low frequency of 1 to 10 Hz by the frequency changing means to make the light source illuminate.

According to a fifth aspect in the lighting device for improving cognitive symptom and depression symptom of the fourth aspect, the lighting device further includes at least one more light source, one of the light sources is made to illuminate by the irradiation switch, and the other light source is made to illuminate by the light-irradiation switch.

### [EFFECT OF THE INVENTION]

According to the present invention, it is possible to sustainably express Homer 1a, and to activate BK-type potassium channels in pyramidal cell of cerebral cortex. As a result, it is possible to improve cognitive symptom and depression symptom.

### [BRIEF DESCRIPTION OF THE DRAWINGS]

Fig. 1 is a configuration diagram showing a room according to an aspect of the present disclosure;
Fig. 2 is a block diagram showing a lighting device for improving cognitive symptom and depression symptom according to another aspect of the present disclosure;
Fig. 3 is a block diagram showing a lighting device for improving cognitive symptom and depression symptom according to the invention;
Fig. 4 is a diagram showing variation in BK channel activation in a depressed model mouse (FS), a mouse (PS) obtained by giving photostimulation to a depressed model mouse, and a non-depressed wild mouse (WT);
Fig. 5 is a diagram showing expression of Homer 1a gene in a depressed model mouse (FS) and a mouse (PS) obtained by giving photostimulation to a depressed model mouse based on a wild mouse (WT) as a reference;
Fig. 6 is a diagram showing a swimming distance of an effectiveness evaluation test carried out by a forced swimming method;
Fig. 7 is a diagram showing immobility time of the effectiveness evaluation test carried out by the forced swimming method;
Fig. 8 is a diagram showing variation in BK channel activation in an Alzheimer disease model mouse (AD), a mouse (PS) obtained by giving photostimulation to an Alzheimer disease model mouse, and a wild mouse (WT) which is not affected by Alzheimer disease;
Fig. 9 is a diagram showing expressions of genes of intracellular protein Homer 1a, intracellular protein Homer 1c, brain-derived neurotrophic factor (BDNF) and BK-type potassium channel (BK);
Fig. 10 is a diagram showing experiment results of an effectiveness evaluation test carried out by Morris water maze method; and
Fig. 11 is a diagram showing experiment results of an effectiveness evaluation test carried out by a fear learning method.

### [EXPLANATION OF SYMBOLS]

- 10: room
- 20: light irradiation device
- 21: light source
- 30: lighting device
- 31: light source
- 32: frequency changing means
- 33: irradiation switch
- 34: light-irradiation switch
- 40: lighting device
- 41: light source
- 42: light source
- 43: irradiation switch
- 44: light-irradiation switch
- 45: high frequency changing means
- 46: low frequency changing means

### [MODE FOR CARRYING OUT THE INVENTION]

In a light irradiation device for improving cognitive symptom and depression symptom according to a first aspect, photostimulation is given to a retina by continuous flashing of a light source of frequency of 1 to 10 Hz. According to this aspect, it is possible to exert an effect equivalent to a depression symptom improving effect obtained by an electroconvusion stimulation device, an effect equivalent to a depression symptom improving effect and an effect equivalent to a cognitive symptom improving effect obtained by a transcranial magnetic stimulation device. It is also possible to utilize these devices also in home environment as compared with an electroconvusion stimulation device and a transcranial magnetic stimulation device which are limited to treatment in clinical institutions as opposed to home environment.

According to a second aspect, in the light irradiation device for improving cognitive symptom and depression symptom of the first aspect, Homer 1a is made to continuously express by the photostimulation, and BK-type potassium channel in pyramidal cell of cerebral cortex is activated. According to this aspect, by continuously giving photostimulation to a retina, it is possible to activate BK-type potassium channel. As a result, it is possible to improve cognitive symptom and depression symptom.

A third aspect provides a room including the light irradiation device for improving cognitive symptom and depression symptom according to the first or second aspect. According to this aspect, it is possible to improve cognitive symptom and depression symptom only by staying in home environment.

A fourth aspect provides a lighting device for improving cognitive symptom and depression symptom including a light source, frequency changing means for changing frequency of the light source, an irradiation switch for changing the frequency to high frequency of 20 to 50 kHz by the frequency changing means to make the light source illuminate, and a light-irradiation switch for changing the frequency to low frequency of 1 to 10 Hz by the frequency changing means to make the light source illuminate. According to this aspect, by continuously giving photostimulation to a retina by operation of the light-irradiation switch, it is possible to activate BK-type potassium channel.

According to the invention, in the lighting device for improving cognitive symptom and depression symptom of the fourth aspect, the lighting device further including at least one more light source, one of the light sources is made to illuminate by the irradiation switch, and the other light source is made to illuminate by the light-irradiation switch. According to this aspect, by continuously giving photostimulation to a retina, it is possible to activate BK-type potassium channel even under bright environment.

### [EMBODIMENTS]

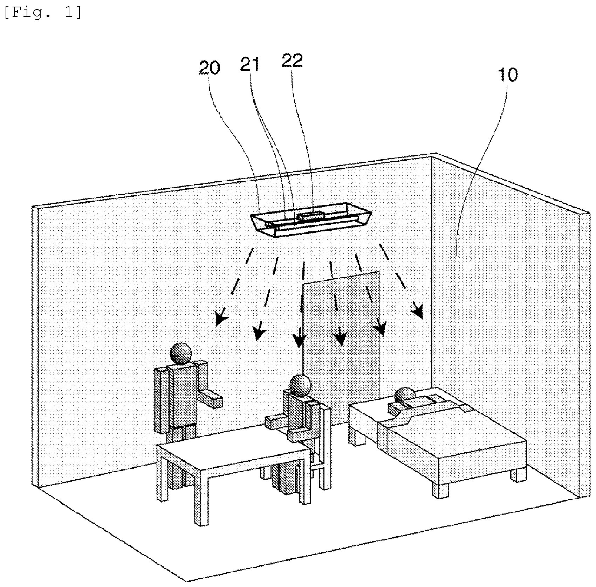

Fig. 1 is a configuration diagram showing a room. A room 10 according to this aspect includes a light irradiation device 20 for improving cognitive symptom and depression symptom. The light irradiation device 20 gives photostimulation to a retina of a person in the room 10 by continuous flashing of a light source 21. Required lighting intensity of the light source 21 is such a level that cells of a retina can sense the light, and it is unnecessary that the lighting intensity is such a level that the person in the room 10 can recognize the light. The continuous flashing of the light source 21 is controlled by a flashing controller 22. The flashing controller 22 carries out flashing by changing frequency of commercial power source to 1 to 10 Hz by changing frequency for example. More preferably, frequency for flashing is 2 to 9 Hz. With flashing of frequency of 1 Hz or less, transmission of synapse is suppressed and inhibitory mechanism strongly acts. With flashing of frequency exceeding 10 Hz, transmission of synapse is enhanced and excitability grows. Flashing of neutral frequency at which inhibition and excitability does not affect is preferable.

The light irradiation device 20 makes Homer 1a continuously express by photostimulation of cyclical flashing, and activates BK-type potassium channel in pyramidal cell of cerebral cortex.

Although the light irradiation device 20 is installed in the room 10 in this aspect, the light irradiation device 20 may have human body attaching means with which the light irradiation device 20 is attached to a human body, and the light irradiation device 20 may be attached to the human body.

Here, as the human body attaching means, there are eyeglasses type attaching means including a frame which forms a rim, a temple and a modern, and cap type attaching means (head mount) which is attached to a head.

It is preferable that the light irradiation device 20 includes the light source 21 and the flashing controller 22 together with the eyeglasses type attaching means and the attaching means. In the eyeglasses type attaching means, lenses or the rim is provided with the light source 21, and the temple or the modern is provided with the flashing controller 22 and a battery. In the cap type attaching means, a flange is provided with the light source 21 or is hanged down from the flange, and a head attaching portion is provided with the flashing controller 22 and the battery.

Instead of the human body attaching means, glass attaching means may be used. That is, the light irradiation device 20 includes the light source 21 composed of LEDs for example, the flashing controller 22, the battery, and the glass attaching means composed of a clip for example, and the glass attaching means may be attached to existing glasses.

Further, a head mount display provided on the light irradiation device 20 may be used as the light source 21.

The human body attaching means may be an eye-mask or goggles covering eyes, or a face mask covering an entire face.

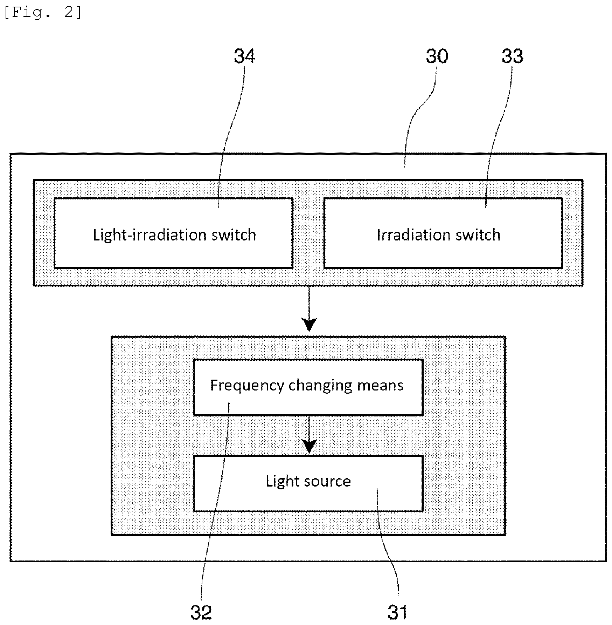

Fig. 2 is a block diagram showing a lighting device for improving cognitive symptom and depression symptom. The lighting device 30 according to this aspect includes a light source 31, frequency changing means 32, an irradiation switch 33 and a light-irradiation switch 34. The frequency changing means 32 changes frequency of the light source. The irradiation switch 33 changes frequency to high frequency of 20 to 50 kHz by the frequency changing means 32, and makes the light source flash. The light-irradiation switch 34 changes frequency to low frequency of 1 to 10 Hz by the frequency changing means 32, and makes the light source flash.

If the irradiation switch 33 is operated, the lighting device 30 of the embodiment illuminates at high frequency at such a level that people do not feel flashing (flicker). If the light-irradiation switch 34 is operated, the lighting device 30 flashes at low frequency at such a level that people feel flashing. At illumination carried out by the operation of the irradiation switch 33, frequency may not be changed, and the lighting device 30 may illuminate at commercial power source frequency.

In this aspect, like the previous aspect, it is preferable that frequency for flashing is 2 to 9 Hz. Here, Homer 1a is continuously expressed by the light-irradiation switch 34, and BK-type potassium channel in pyramidal cell of cerebral cortex is activated.

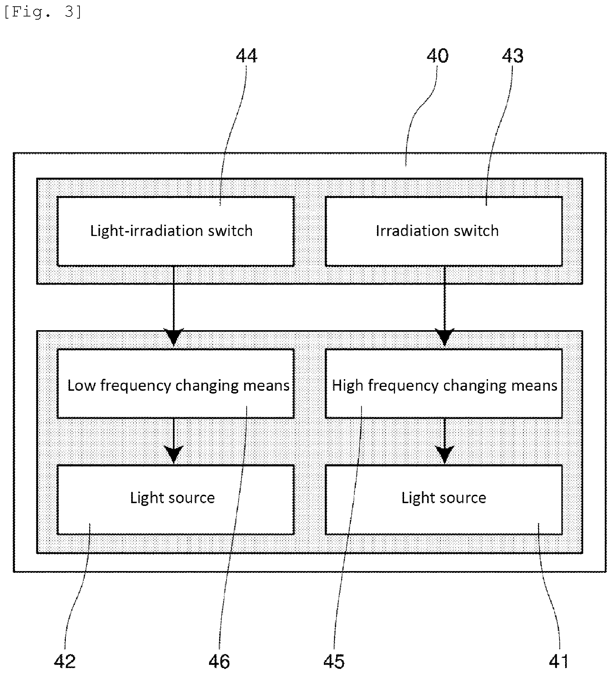

Fig. 3 is a block diagram showing a lighting device for improving cognitive symptom and depression symptom according to the invention.

A lighting device 40 of this embodiment includes a plurality of light sources 41 and 42. The light source 41 is made to illuminate by an irradiation switch 43, and the light source 42 is made to illuminate by a light-irradiation switch 44.

The light source 41 illuminates at high frequency of 20 to 50 kHz by high frequency changing means 45. The light source 42 illuminates at low frequency of 1 to 10 Hz by low frequency changing means 46.

If the irradiation switch 43 is operated, the lighting device 40 of the embodiment illuminates at high frequency at such a level that people do not feel flashing (flicker). If the light-irradiation switch 44 is operated, the lighting device 40 flashes at low frequency at such a level that people feel flashing. In this embodiment, the operation of the irradiation switch 43 and the operation of the light-irradiation switch 44 can be carried out at the same time. That is, even under bright environment by the irradiation switch 43, it is possible to continuously give photostimulation to a retina by the light-irradiation switch 44 and thus, it is possible to activate BK-type potassium channel.

In this embodiment also, like the previous aspects, it is more preferable that frequency for flashing is 2 to 9 Hz.

At illumination carried out by operation of the irradiation switch 43, the lighting device 40 may not be provided with the high frequency changing means 45, and may illuminate at commercial power source frequency.

Experiment results of mouse by photostimulation will be described below.

As photostimulation, a light source of 300 Lx is made to flash at frequency of 2 Hz. The flashing is continuously carried out for six hours a day for four weeks.

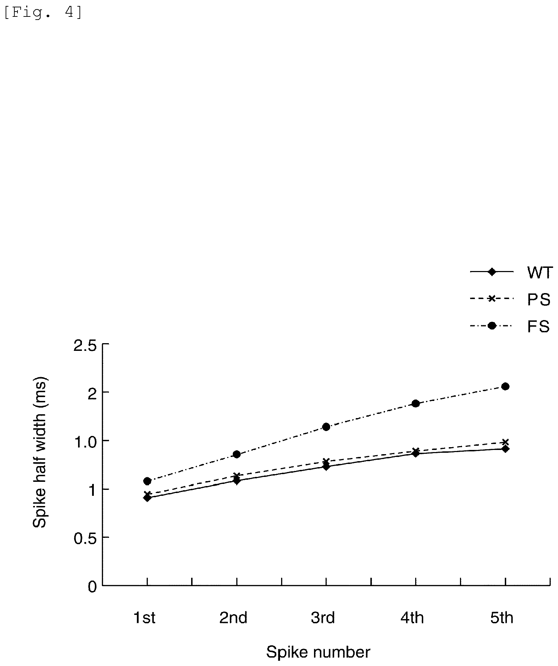

Fig. 4 shows experiment results showing variation in BK channel activation in a depressed model mouse (FS), a mouse (PS) obtained by giving photostimulation to a depressed model mouse, and a non-depressed wild mouse (WT). The lower a numeric value is, the higher the activation is. In the mouse (PS) obtained by giving photostimulation to a depressed model mouse, it is found that BK channel is recovered to the same level as the wild mouse (WT).

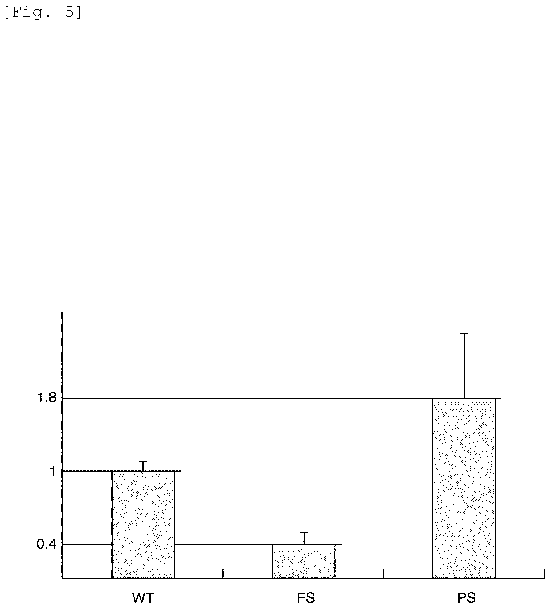

Fig. 5 shows experiment results in which expressions of genes of intracellular protein Homer 1a are compared with each other.

In Fig. 5, the wild mouse (WT) is shown as a reference, and expressions of genes of the depressed model mouse (FS) and the mouse (PS) obtained by giving photostimulation to a depressed model mouse are shown in comparison.

It is found that in the depressed model mouse (FS), expression of gene of intracellular protein Homer 1a is reduced to about 0.4 as compared with the wild mouse (WT), whereas in the mouse (PS) obtained by giving photostimulation to a depressed model mouse, expression of gene of intracellular protein Homer 1a is increased to about 1.8 times.

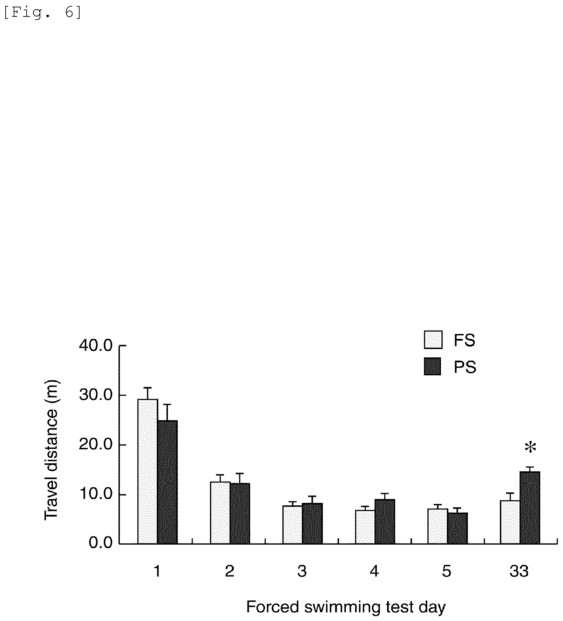

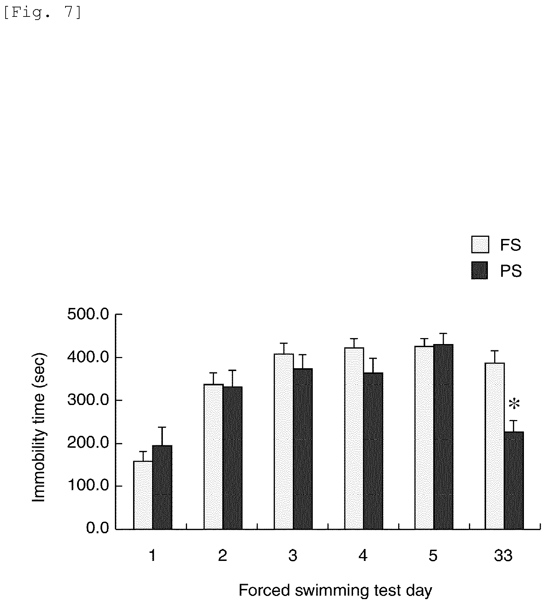

Figs. 6 and 7 shows experiment results of effectiveness evaluation tests carried out by a forced swimming method.

The depressed model mouse (FS) and the mouse (PS) obtained by giving photostimulation to a depressed model mouse were made to forcibly swim for five days, and behavior of the depressed model mouse (FS) and behavior of the mouse (PS) after four weeks were evaluated.

Fig. 6 shows swimming distances, and Fig. 7 shows immobility time.

As shown in Fig. 6, no significant difference can be recognized between a swimming distance of the depressed model mouse (FS) after four weeks and a swimming distance of the depressed model mouse (FS) on a fifth day of the forced swimming test period. On the other hand, a significant extension was recognized between a swimming distance of the mouse (PS) obtained by giving photostimulation to a depressed model mouse after four weeks and a swimming distance of the mouse (PS) on a fifth day of the forced swimming test period.

As shown in Fig. 7, no significant difference can be recognized between immobility time of the depressed model mouse (FS) after four weeks and immobility time of the depressed model mouse (FS) on a fifth day of the forced swimming test period. On the other hand, a significant reduction was recognized between immobility time of the mouse (PS) obtained by giving photostimulation to a depressed model mouse after four weeks and immobility time of the mouse (PS) on a fifth day of the forced swimming test period.

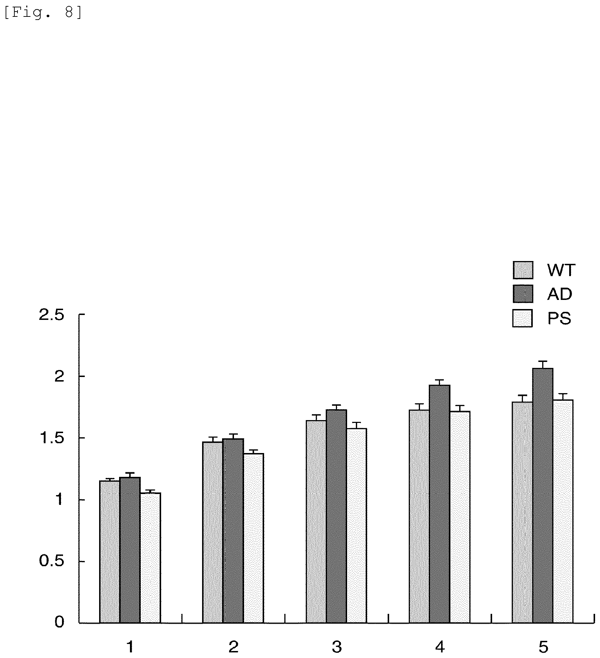

Fig. 8 shows experiment results showing variation in BK channel activation in an Alzheimer disease model mouse (AD), a mouse (PS) obtained by giving photostimulation to an Alzheimer disease model mouse, and a wild mouse (WT) which is not affected by Alzheimer disease. The lower a numeric value is, the higher the activation is. In the mouse (PS) obtained by giving photostimulation to an Alzheimer disease model mouse, it is found that BK channel is recovered to the same level as the wild mouse (WT).

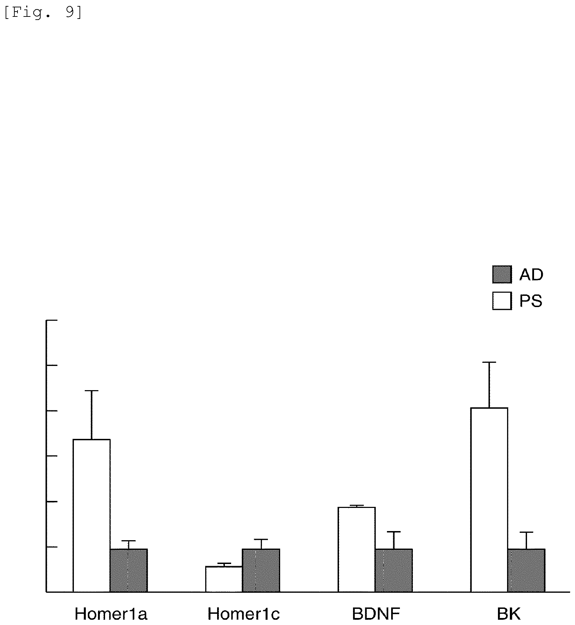

Fig. 9 shows experiment results showing comparison of expressions of genes of intracellular protein between Homer 1a, intracellular protein Homer 1c, brain-derived neurotrophic factor (BDNF) and BK-type potassium channel (BK) .

In Fig. 9, the Alzheimer disease model mouse (AD) is shown as a reference, expressions of genes, by a quantitative RT-PCR method, of the Alzheimer disease model mouse (AD) and the mouse (PS) obtained by giving photostimulation to an Alzheimer disease model mouse are shown in comparison.

It is found that expression of gene of the intracellular protein Homer 1a of the mouse (PS) obtained by giving photostimulation to an Alzheimer disease model mouse is increased by about 3.2 times as compared with the Alzheimer disease model mouse (AD).

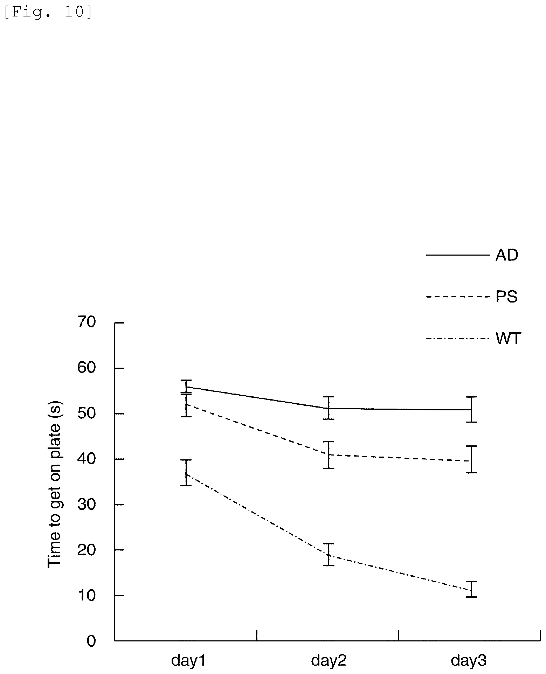

Fig. 10 shows experiment results of an effectiveness evaluation test carried out by Morris water maze method.

Memory learning was conducted for three days for the Alzheimer disease model mouse (AD), the mouse (PS) obtained by giving photostimulation to an Alzheimer disease model mouse, and the wild mouse (WT) which is not affected by Alzheimer disease, and results were evaluated.

Although the mouse (PS) obtained by giving photostimulation to an Alzheimer disease model mouse is inferior in memory ability to the wild mouse (WT) which is not affected by Alzheimer disease, slight improvement of the mouse (PS) was recognized as compared with the Alzheimer disease model mouse (AD).

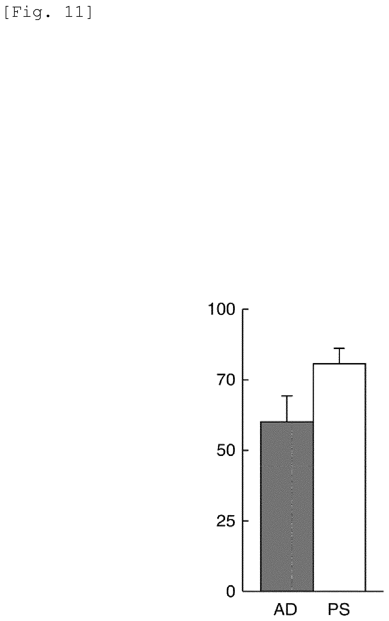

Fig. 11 shows experiment results of an effectiveness evaluation test carried out by a fear learning method, and shows a ratio of cowering time for five minutes.

Cowering time of the mouse (PS) obtained by giving photostimulation to an Alzheimer disease model mouse is longer than that of the Alzheimer disease model mouse (AD), and it is found that learning and memory effects are enhanced.

The photostimulation of the present invention is closer to noninvasive than minimally-invasive, and the photostimulation can be utilized in home. The photostimulation does not have side effect, and utilizes physiological process, i.e., retina stimulation. Further, since the light irradiation device is used, a special doctor such as not only a psychiatrist but also an anesthesiologist are not required, and user's high expertise is not required.

In an animal experiment, the same effect as those of the repetitive transcranial magnetic stimulation and the medical agent (imipramine) could be obtained. Therefore, it was discovered in the animal experiment that the electroconvulsive therapy, the repetitive transcranial magnetic stimulation and the imipramine have mechanism in common.

In the present invention, extremely physiological process, i.e., photostimulation for a retina is utilized. Further, the invention utilizes neurobiological action, i.e., utilizes expression of cerebral cortex nerve intracellular protein Homer 1a. A common point is through expression enhancement of Homer 1a.

The present invention can be applied not only to depression but also to Alzheimer disease. Neurobiological mechanisms of depression and Alzheimer disease including expression of Homer 1a are the same. Hence, the present invention can be used not only for treatment of each of depression and Alzheimer disease, but also for common composite treatments for both of them.

If the present invention is compared with high lighting intensity light therapy, the invention can be utilized irrespective of seasonal and nonseasonal, and the invention is not limited to seasonal use. The invention does not depend on action mechanism which is assumed in the high lighting intensity light therapy. In the invention, flashing light of low lighting intensity is used instead of continuous light of high lighting intensity. As a result, an excellent effect equivalent to antidepressants such as imipramine is obtained by the animal experiment.

An effect of photostimulation of low lighting intensity is denied in nonseasonal depression but in the invention, low lighting intensity photostimulation which is equal to or lower than it is effective. The low lighting intensity photostimulation in the present invention is effective not only for seasonal depression, but can widely be applied to general depression, Alzheimer disease and a combination example thereof.

### [INDUSTRIAL APPLICABILITY]

The treatment device for cognitive symptom and depression symptom of the present invention can be used not only in clinical institutions but also in home environment of standard home.

## Claims

1. A light irradiation device (40) for improving cognitive symptom and depression symptom comprising:
a first light source (42);
a low frequency changing means (46); a light-irradiation switch (44) connected to the low frequency changing means (46) configured for changing frequency of the first light source (42) to make it flash at a low frequency of 1 to 10 Hz;
a second light source (41);
a high frequency changing means (45); and
an irradiation switch (43) connected to the high frequency changing means (45) configured for changing frequency of the second light source (41) to make it flash at a high frequency of 20 to 50 kHz;
wherein photostimulation continuously given to a retina by continuous flashing of said light sources (41, 42) makes Homer 1a to continuously express and activates BK-type potassium channel in pyramidal cell of cerebral cortex even under bright environment.

## Patentansprüche

1. Lichtbestrahlungsvorrichtung (40) zur Besserung eines kognitiven Symptoms und eines Depressionssymptoms, umfassend:
eine erste Lichtquelle (42),
eine Frequenzänderungseinrichtung (46) für niedrige Frequenz, einen Lichtbestrahlungs-Schalter (44), der mit der Frequenzänderungseinrichtung (46) für niedrige Frequenz verbunden ist, welche für eine Änderung der Frequenz der ersten Lichtquelle (42) ausgelegt ist, um diese bei einer niedrigen Frequenz von 1 bis 10Hz blitzen zu lassen,
eine zweite Lichtquelle (41),
eine Frequenzänderungseinrichtung (45) für hohe Frequenz und einen Bestrahlungs-Schalter (43), der mit der Frequenzänderungseinrichtung (45) für hohe Frequenz verbunden ist, welche für eine Änderung der Frequenz der zweiten Lichtquelle (41) ausgelegt ist, um diese bei einer hohen Frequenz von 20 bis 50kHz blitzen bzw. blinken zu lassen,
wobei eine einer Retina durch das kontinuierliche Blitzen der Lichtquellen (41, 42) kontinuierlich vermittelte Fotostimulation ein kontinuierliches Exprimieren von Homer 1a bewirkt und den Kaliumkanal des BK-Typs in der Pyramidenzelle der Großhirnrinde sogar in heller Umgebung aktiviert.

## Revendications

1. Dispositif de rayonnement de lumière (40) permettant d'améliorer des symptômes cognitifs et des symptômes de dépression, comprenant :
une première source de lumière (42) ;
un moyen de variation de basse fréquence (46) ;
un commutateur de rayonnement de lumière (44) raccordé au moyen de variation de basse fréquence (46) conçu pour faire varier la fréquence de la première source de lumière (42) de façon à la faire clignoter à une basse fréquence de l'ordre de 1 à 10 Hz ;
une seconde source de lumière (41) ;
un moyen de variation de haute fréquence (45) ; et
un commutateur de rayonnement (43) raccordé au moyen de variation de haute fréquence (45) conçu pour faire varier la fréquence de la seconde source de lumière (41) de façon à la faire clignoter à une haute fréquence de l'ordre de 20 à 50 kHz ;
dans lequel la photostimulation conférée en continu à une rétine par le clignotement en continu desdites sources de lumière (41, 42) induit l'expression continue de Homer 1a et l'activation des canaux potassiques de type BK dans des cellules pyramidales du cortex cérébral, même dans un environnement lumineux.
